# DEMANDE DE BREVET EUROPEEN

(11) **EP 2 921 471 A1**
(43) Date de publication de la demande: **23.09.2015**
(21) Numéro de dépôt: 15157900.0
(22) Date de dépôt: 05.03.2015
(51) Int. Cl.: C07C 51/47, C07C 57/02

(54) **Procédé d'extraction de l'acide aconitique à partir de produits issus de l'industrie de la canne à sucre**

(30) Priorité: 18.03.2014 FR 1452245
(71) Demandeur: eRcane, 97494 Sainte-Clotilde cedex (FR); Institute National Polytechnique de Toulouse, B.P. 34 038 31029 Toulouse Cedex 4 (FR); Université de La Réunion, 97744 Saint Denis cedex 9 (FR)
(72) Inventeur: Petit, Arnaud, 97490 Sainte Clotilde (FR); Wu-Tiu-Yen, Jenny, 97400 Saint Denis (FR); Pislor, Emilie, 31320 Castanet Tolosan (FR); Pontalier, Pierre-Yves, 31500 Toulouse (FR); Albet, Joël, 31750 Escalquens (FR); Hoareau, Wiliam, 97480 Saint Joseph (FR); Corcodel, Laurent, 10000 Troyes (FR)
(74) Mandataire: Pontet Allano & Associes

(57) **Abrégé**

La présente invention concerne un procédé d'extraction de l'acide aconitique à partir de produits issus de l'industrie de la canne à sucre, ledit procédé comprenant une étape de l'élution de l'acide aconitique fixé sur une résine anionique et une étape de la purification de l'éluat.

## Description

La présente invention concerne un procédé d'extraction de l'acide aconitique à partir de produits issus de l'industrie de la canne à sucre.

L'acide aconitique, ayant un fort potentiel de valorisation industrielle, est un acide organique synthétisé dans les cannes à sucre lors de l'assimilation du potassium et se trouve dans les différents produits de l'industrie canne - sucre - alcool, tels que le jus d'extraction, le jus clair, le sirop, la masse-cuite, l'égout de 1^{ère} cristallisation, l'égout de 2^{ème} cristallisation, l'égout de 3^{ème} cristallisation, et notamment dans trois produits principaux : la mélasse, la vinasse, et le jus clair.

L'acide aconitique (acide 1-propène-1,2,3 tricarboxylique) est un triacide aliphatique insaturé de formule C₆H₆O₆ qui existe naturellement sous deux formes isomères (*cis* ou *trans*)*.*

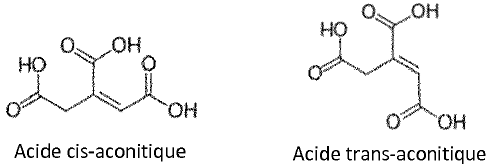

### État de l'art de l'extraction de l'acide aconitique

Il existe différentes méthodes d'extraction des acides organiques à partir de matrices d'origines végétales : la précipitation, l'extraction liquide/liquide, l'échange d'ions, les procédés membranaires. Cependant, les projets de recherche visant à appliquer ces méthodes à l'acide aconitique soit ne respectent pas les principes de la chimie verte, soit n'ont pas permis d'obtenir une pureté supérieure à 22% et ne correspondent pas aux exigences industrielles.

Ainsi, il est possible d'obtenir une poudre d'acide aconitique avec une pureté élevée, par des méthodes très polluantes, telles que l'extraction liquide-liquide en utilisant des solvants toxiques. Il est nécessaire de développer une méthode plus écologique d'extraction d'acide aconitique de haute pureté (supérieure à 90%) et permettant de mettre en oeuvre les produits issus de l'industrie de la canne à sucre.

### Précipitation

La précipitation de l'acide aconitique contenu dans des extraits de plantes, tels que la mélasse de canne à sucre, est effectuée par l'introduction de sels alcalins.

Des études ont été menées sur la récupération d'acides organiques, notamment l'acide aconitique, à partir de produits de l'industrie sucrière. En 1991 Hanine et al. (Hanine et al., 1991, Int. Sugar J. 93 (1115): 232-237) ont étudié les différents paramètres influençant la précipitation de l'acide aconitique en aconitate de tricalcium par ajout de CaCl₂. Il a d'ailleurs été démontré que l'apport d'une quantité de calcium plus importante que la seule valeur stoechiométrique permet d'augmenter le rendement de précipitation de l'acide. D'autre part, plus la solution d'acide aconitique est concentrée, meilleur sera le rendement de précipitation : Le rendement de précipitation de l'acide aconitique au CaCl₂ en quantité stoechiométrique n'excède pas 50% lorsque la concentration en acide aconitique est inférieure à 25 g/L, et avoisine les 78% pour une solution concentrée à 40 g/L.

Les inconvénients majeurs de la précipitation sont :
- La nécessité de dissocier l'aconitate de calcium de l'acide sulfurique, ce qui engendre de fortes quantités de boues à retraiter. L'acide sulfurique présent dans la solution d'acide aconitique abaisse d'autant plus la pureté de la solution et engendre une très forte acidité.
- La co-précipitation d'autres composés (colorants, acides organiques, sucres) qui affecte aussi la pureté globale de l'acide aconitique obtenu et qui nécessite des étapes de purifications supplémentaires.

### Extraction liquide/liquide

Une étude antérieure a montré qu'il est possible de récupérer et de séparer certains acides carboxyliques dans des solutions diluées. Le tributylphosphate est un bon solvant d'extraction (Malmary et al, 2000, J. Chem. Tech. Biotechnol. 75 (12): 1169-1173).

L'extraction liquide/liquide a été expérimentée à l'échelle du laboratoire pour extraire l'acide aconitique à partir de mélasse de canne à sucre (US 2,712,552).

Les premiers essais d'extraction ont été effectués avec la méthylisobutylcétone (MIBK) et la méthyléthylcétone (MEK) comme solvant d'extraction (Regna and Bruins, 1956, Ind. Eng. Chem. 48 (8):1268-1277). L'acétate d'éthyle a également été testé (Azzam and Radwan, 1986, Fette, Seifen, Anstrichmittel, 88 (3): 97-99). L'utilisation du tributylphosphate (TBP) et de la triisooctylamine (TIOA) ont permis d'obtenir de meilleurs rendements d'extraction (Monteil, 1990, "Extraction de l'acide aconitique", DEA, INP-ENSCT).

L'extraction des acides carboxyliques par cette technique est rendue difficile par le caractère hydrophile de l'acide qui accentue l'affinité de l'acide pour la phase aqueuse. De plus, la présence de plusieurs groupes carboxyles et hydroxyles sur la chaîne carbonée amplifie cette affinité. Cette technique d'extraction nécessite alors une grande énergie d'interaction entre les molécules du solvant et ceux de l'acide.

Pislor et al. (2009, 2nd International Congress on Green Process Engineering, 2nd European Process Intensification Conference, 14-17 June 2009) ont obtenu de bons rendements d'extraction de l'acide aconitique dans la mélasse et la vinasse acidifiées (pH=2) avec un mélange de solvant tributylphosphate/n-dodécane (60/40). Cependant, ce procédé ne permet pas d'extraire efficacement l'acide aconitique dans une solution de pH=4-5, comme c'est le cas dans la mélasse ou dans la vinasse naturelle, alors qu'une acidification de la vinasse nécessiterait un traitement supplémentaire qui augmenterait le coût de production de l'acide aconitique.

Par ailleurs, cette méthode nécessite l'utilisation de solvants organiques qui peuvent nuire à l'environnement.

### Echange d'ions

La séparation par chromatographie par résine échangeuse d'ions a été utilisée dans la désacidification des jus de fruit (Vera et al., 2003, J. Food Eng., 57 (2): 199-207) et la purification des acides organiques de milieux fermentés (Moldes et al., 2003, Bioproc. Biosyst. Eng., 25 (6): 357-363).

En 2006, Saska et Zapata (Saska and Zapata, 2006, Int. Sugar J., 108 (1288): 203-208) ont étudié la possibilité d'extraire l'acide aconitique à partir de produits issus de canne à sucre en utilisant une résine fortement anionique et une résine adsorbante non ionique. La résine échangeuse fortement anionique permet la récupération de l'acide aconitique d'une solution ramenée à pH ≈ 2,8.

Pislor *et al.* ont décrit une méthode d'extraction de l'acide aconitique dans une solution à pH=5 en utilisant une résine fortement anionique sous forme de chlorure (Amberlite® IRA 900) et un éluant basique (Pislor et al., 2009, 2nd International Congress on Green Process Engineering, 2nd European Process Intensification Conference, 14-17 June 2009).

Cependant, cette méthode ne permet d'obtenir un bon rendement d'extraction que lorsque l'acide aconitique est en faible concentration dans une solution, et ne convient pas à une extraction à l'échelle industrielle.

### Procédés membranaires

La séparation de l'acide aconitique peut également se faire par des procédés membranaires, qui permettent la récupération de l'acide aconitique par passage sélectif d'un ou de plusieurs composés à travers une membrane. Cette technique est induite par des forces motrices pouvant être de nature physique (gradient de pression) ou chimique (gradient de concentration en soluté), et dépend des membranes utilisées, notamment de leurs sélectivités.

Parmi les procédés membranaires, la nanofiltration et l'osmose inverse semblent être les techniques membranaires les plus intéressantes pour séparer ou concentrer les acides organiques (Timmer et al., 1994, J. Membr. Sci.,92 (2) : 185-197).

Cependant, ces techniques de filtration membranaire dépendent majoritairement de l'état d'ionisation des molécules et donc du pH de la solution.

Etant donné que les procédés d'extraction de l'acide aconitique connus dans l'art antérieur présentent des inconvénients techniques qui, soit empêchent l'augmentation du rendement ou de la pureté de l'extraction, soit sont polluants pour l'environnement lorsque le procédé est mis en oeuvre à grande échelle, les industries de la canne à sucre ont besoin d'un nouveau procédé d'extraction de l'acide aconitique avec un meilleur rendement, mais permettant également d'obtenir une grande pureté, et satisfaisant à l'exigence écologique.

La présente invention permet de répondre à ces besoins et a pour objet de proposer un procédé d'extraction de l'acide aconitique à partir des produits issus de l'industrie de la canne à sucre.

Le procédé de l'invention comprend une étape d'échange anionique sur une résine, suivie par une étape de purification par nanofiltration.

Le procédé de l'invention comprend :
(i) la mise en contact d'une solution de l'un desdits produits contenant l'acide aconitique avec une résine faiblement ou fortement anionique sous forme de base libre ou préalablement conditionnée sous forme d'un contre-anion,
(ii) l'élution de l'acide aconitique à partir de ladite résine pour obtenir un éluat contenant l'acide aconitique, et
(iii) la purification de l'éluat par une nanofiltration sur des membranes ayant un seuil de rétention de 100 à 1000 g/mol, éventuellement en mode diafiltration, pour obtenir un éluat purifié contenant l'acide aconitique,
ladite résine comportant des groupements ammonium quaternaire et éventuellement des groupements amines tertiaires et ayant une granulométrie comprise entre 200 et 2000 µm.

Le procédé de l'invention permet de produire l'acide aconitique sous forme cristalline ayant une pureté supérieure à 90%.

Au sens de la présente invention, l'acide aconitique extrait par le présent procédé est un mélange d'acide *cis*-aconitique et d'acide *trans-*aconitique.

L'acide cis-aconitique ou l'acide trans-aconitique peuvent être séparés selon toutes méthodes de séparation connues de l'homme du métier.

Dans le cadre de la présente invention, les produits contenant l'acide aconitique issus de l'industrie de la canne à sucre sont choisis dans le groupe comprenant le jus d'extraction, le jus clair, le sirop, l'égout de 1^{ère} cristallisation, l'égout de 2^{ème} cristallisation, l'égout de 3^{ème} cristallisation ou mélasse, les vins de fermentation obtenus à partir des produits précédemment cités et les vinasses obtenues après distillation de ces vins.

On entend par « jus clair », le jus obtenu après l'élimination des matières en suspension et de certaines impuretés solubles présentes dans le « jus d'extraction ».

On entend par « sirop », le jus obtenu après concentration par évaporation du « jus clair ».

On entend par « égout de ... cristallisation », la fraction liquide baignant les cristaux de sucres et séparée de ceux-ci par centrifugation.

On entend par « mélasse », autrement dit « égout de 3^{ème} cristallisation », le sirop résiduel obtenu après trois répétitions de l'étape de cristallisation de sucre et de l'étape de la séparation des cristaux de sucre.

### Etape d'échange anionique

On entend par « résine faiblement ou fortement anionique », une résine comportant des groupes fonctionnels faiblement ou fortement positifs, lesquels sont liés à la résine par liaison covalente, et des contre-anions échangeables ou des bases libres.

Dans le cadre de la présente invention, une résine faiblement anionique est une résine comportant des groupes fonctionnels faiblement positifs, tels qu'un groupement d'amine tertiaire, alors qu'une résine fortement anionique est une résine comportant des groupes fonctionnels fortement positifs, tels qu'un groupement d'ammonium quaternaire.

Les contre-anions échangeables dans une résine faiblement ou fortement anionique peuvent être un contre-anion monovalent, divalent ou trivalent issu d'un acide fort, plus particulièrement : de l'acide sulfurique, de l'acide chlorohydrique, de l'acide nitrique, de l'acide permanganique, de l'acide perchlorique, de l'acide iodhydrique, de l'acide bromhydrique, de l'acide benzènesulfonique, de l'acide trichloroacétique, de l'acide thiosulfurique, de l'acide iodique, de l'acide thiocyanique, de l'acide sulfamique ou de l'acide oxalique.

La résine utilisée dans le cadre de l'invention peut être un polymère éventuellement réticulé, tel que polystyrène, polyacrylamide, polyacrylate, ou un copolymère, tel que le copolymère polystyrène/divinylbenzène.

Conformément à l'invention, une résine faiblement anionique susceptible d'être utilisée dans l'invention est soit une résine de polystyrène réticulé comportant des amines tertiaires et conditionnée sous forme de sulfate et avec une granulométrie comprise entre 200 et 2000 µm, en particulier la résine LEWATIT® S4528 ou la résine LEWATIT® MP62 ; soit une résine de copolymère polystyrène/divinylbenzène comportant les amines tertiaires conditionnées sous forme de sulfate et ayant une granulométrie comprise entre 200 et 2000 µm, en particulier la résine DOWEX® 66.

Conformément à l'invention, une résine fortement anionique susceptible d'être utilisée dans l'invention est soit une résine de copolymère polystyrène/divinylbenzène comportant les ammoniums quaternaires conditionnée sous forme chlorure et ayant une granulométrie comprise entre 200 et 2000 µm, en particulier la résine Amberlite® IRA 900 CI ou la résine Dowex® Marathon MSA; soit une résine de polyacrylate réticulé comportant les ammoniums quaternaires conditionnée sous forme chlorure et ayant une granulométrie comprise entre 200 et 2000 µm, en particulier la résine Amberlite® 958.

L'utilisation d'une résine faiblement ou fortement anionique permet une extraction sélective des acides organiques vis-à-vis des espèces neutres ou chargées positivement. De plus, l'utilisation d'une résine faiblement ou fortement anionique permet une extraction sélective de l'acide aconitique vis-à-vis d'autres acides organiques initialement présent dans le coproduit tels que l'acide malique, l'acide acétique, l'acide lactique, l'acide succinique, dans des conditions de pH judicieusement choisies par l'homme du métier.

Dans un mode de réalisation particulier de l'invention, une solution contenant l'acide aconitique est mise en contact avec la résine avec un débit de fixation de 0,5 à 20 BV/h, notamment de 1 à 5 BV/h.

L'élution de l'acide aconitique à partir de résine peut être réalisée soit par une solution d'acide, soit par une solution de base.

Dans un mode de réalisation particulier, l'étape d'élution du procédé de l'invention est réalisée par une solution d'acide à une concentration comprise entre 0,1 et 6 N, ledit acide étant choisi parmi l'acide sulfurique ou l'acide chlorhydrique.

Dans un mode de réalisation plus particulier, l'élution de l'acide aconitique selon le procédé de l'invention est réalisée par de l'acide sulfurique, lorsque la résine utilisée est conditionnée sous forme sulfate. En effet, l'élution par l'acide sulfurique permet de coupler l'élution et la régénération. Elle permet de collecter des fractions à fortes concentrations en acide aconitique.

Dans un autre mode de réalisation particulier, l'étape d'élution est réalisée par une solution de base à concentration comprise entre 0,1 et 6 N, ladite base étant choisie parmi l'hydroxyde de sodium ou l'hydroxyde de potassium.

Dans un mode de réalisation plus particulier, l'élution de l'acide aconitique selon le procédé de l'invention est mise en oeuvre par la soude, lorsque la résine utilisée est une résine faiblement anionique dont les groupements fonctionnels sont des amines tertiaires.

Dans un mode de réalisation particulier de l'invention, le débit d'élution de l'acide aconitique à partir de la résine est de 0,5 à 10 BV/h (Beda Volume par heure), préférablement de 0,5 à 5 BV/h, car un débit faible, notamment de 0,5 à5 BV/h, favorise la réaction d'échange d'anions et permet d'obtenir un extrait plus concentré, puisque le volume nécessaire à l'élution est réduit.

### Etape de purification

Conformément à l'invention, l'étape de la purification de l'éluât contenant l'acide aconitique est mise en oeuvre par une nano filtration, ce qui permet d'obtenir un éluât purifié contenant l'acide aconitique.

Après la nano filtration, l'acide aconitique sous forme non chargée ou monovalente se trouve dans le permet, alors que sont retenus dans le retentât les molécules à haut poids moléculaire, par exemple les colorants, ainsi que les anions divalents, tels que les ions sulfates.

On entend par « retentât », la fraction composée de molécules retenues par la membrane de nano filtration ; par « permet » la fraction composée de molécules qui passent à travers la membrane de nano filtration.

Dans le cadre de la présente invention, la nano filtration est mise en oeuvre par des membranes ayant un seuil de rétention de 100 à 1000 g/mol, plus particulièrement de 150 à 600 g/mol.

Dans un mode de réalisation plus particulier, l'étape de purification de l'acide aconitique selon le procédé de l'invention est mise en oeuvre par une nano filtration en mode diafiltration sur des membranes ayant un seuil de rétention de 100 à 1000 g/mol.

Au sens de l'invention, la diafiltration peut être mise en oeuvre en mode continu ou discontinu. On entend par « diafiltration continue », une filtration pour laquelle l'eau est ajoutée en continu dans le rétentat pour compenser le débit de perméat. On entend par « diafiltration discontinue », une filtration pour laquelle est réalisée une série de dilution de rétentat et de concentration par filtration à son volume initial.

Selon un mode de réalisation particulier, le procédé d'extraction de l'invention comprend en outre une étape de clarification du produit contenant l'acide aconitique avant l'étape de mise en contact dudit produit avec la résine faiblement ou fortement anionique, afin d'éliminer les matières en suspension présentes dans ce produit et d'éviter un colmatage du lit de résine échangeuse d'ions.

La clarification d'un produit contenant l'acide aconitique selon l'invention est réalisée par filtration ou centrifugation.

Plus particulièrement, l'étape de clarification selon l'invention peut être mise en oeuvre par une filtration sur une membrane de microfiltration ayant une porosité de 0,1 à 10 µm, éventuellement suivie par une filtration sur des membranes de ultrafiltration ayant une porosité de 1 à 100 nm, ce qui permet d'éliminer à la fois les matières en suspension et certaines macromolécules, comme les colorants, qui pourraient limiter l'efficacité de la fixation de l'acide aconitique.

Dans un mode de réalisation plus particulier, le procédé de l'invention comprend :
(i) la clarification d'une solution d'un produit issu de l'industrie de la canne à sucre pour obtenir une solution clarifiée contenant l'acide aconitique, et
(ii) la mise en contact de ladite solution clarifiée avec une résine faiblement ou fortement anionique sous forme de base libre ou préalablement conditionnée sous forme d'un contre-anion,
(iii) l'élution de l'acide aconitique à partir de ladite résine pour obtenir un éluât contenant l'acide aconitique, et
(iv) la purification de l'éluat obtenu dans l'étape (iii) par une nanofiltration sur des membranes ayant un seuil de rétention de 100 à 1000 g/mol, éventuellement en mode diafiltration.

Selon un autre mode de réalisation particulier, le procédé d'extraction de l'invention comprend en outre une étape de concentration de l'acide aconitique en aval de la purification de l'éluat.

Dans le cadre de l'invention, la concentration de l'éluat purifié est mise en oeuvre par osmose inverse et/ou par nanofiltration, éventuellement suivie par évaporation sous vide.

Selon les conditions de pH auquel on utilise une membrane de nanofiltration, ladite membrane sera utilisée soit pour purifier une solution d'acide aconitique, soit pour la concentrer. L'homme du métier saura à partir de ses connaissances générales déterminer les conditions ad hoc.

Dans le mode de purification par nanofiltration, l'acide aconitique se trouve dans le perméat.

En revanche, l'acide aconitique se trouve dans le concentrat, lorsque la nanofiltration est mise en oeuvre pour la concentration.

Dans le cadre de la présente invention, après cette étape, la concentration de l'acide aconitique dans l'éluat purifié atteint à une concentration nécessaire pour la cristallisation.

Un autre mode de réalisation particulier de l'invention concerne un procédé d'extraction comprenant en outre une étape de décoloration en aval de la clarification d'un produit issu de l'industrie de la canne à sucre contenant l'acide aconitique, pour obtenir une solution décolorée contenant l'acide aconitique.

La décoloration peut être mise en oeuvre soit sur la solution clarifiée obtenue après la clarification d'une solution d'un produit issu de l'industrie de la canne à sucre, soit sur l'éluat obtenu dans l'étape d'élution, soit sur l'éluat purifié obtenu dans l'étape de purification, soit sur le concentrat obtenu dans l'étape de concentration.

La décoloration peut être effectuée par n'importe quelle technique connue de l'homme du métier, telle que par un adsorbant ou par filtration. L'adsorbant susceptible d'être utilisé pour la décoloration du concentrat peut être tout absorbant connu de l'homme du métier, tel qu'un charbon actif.

Lorsque le concentrat à décolorer est sous forme de sirop ou solide, ledit concentrat peut être dissous ou dilué dans l'eau avant la décoloration.

Après la décoloration, l'adsorbant, notamment le charbon actif peut être retiré par filtration, qui peut être réalisée en 2 étapes comportant une filtration avec un filtre ayant un seuil de rétention élevé ou une centrifugation suivie par une filtration avec un filtre ayant un seuil de rétention plus bas. Toutes ces techniques font partie des connaissances générales de l'homme du métier.

Dans un mode de réalisation particulier, le procédé de l'invention comprend :
(i) la clarification d'une solution d'un produit issu de l'industrie de la canne à sucre pour obtenir une solution clarifiée contenant l'acide aconitique
(ii) la décoloration de ladite solution clarifiée obtenue dans l'étape (i) pour obtenir une solution décolorée contenant l'acide aconitique,
(iii) la mise en contact de ladite solution décolorée avec une résine faiblement ou fortement anionique sous forme de base libre ou préalablement conditionnée sous forme d'un contre-anion,
(iv) l'élution de l'acide aconitique à partir de ladite résine pour obtenir un éluât contenant l'acide aconitique,
(v) la purification de l'éluat par une nanofiltration sur des membranes ayant un seuil de rétention de 100 à 1000 g/mol, éventuellement en mode diafiltration, pour obtenir un éluat purifié contenant de l'acide aconitique, et
(vi) la concentration de l'éluat purifié pour obtenir un concentrat contenant l'acide aconitique.

Dans un autre mode de réalisation particulier, le procédé de l'invention comprend :
(i) la clarification d'une solution d'un produit issu de l'industrie de la canne à sucre pour obtenir une solution clarifiée contenant l'acide aconitique, et
(ii) la mise en contact de ladite solution clarifiée avec une résine faiblement ou fortement anionique sous forme de base libre ou préalablement conditionnée sous forme d'un contre-anion,
(iii) l'élution de l'acide aconitique à partir de ladite résine pour obtenir un éluât contenant l'acide aconitique,
(iv) la décoloration de l'éluat obtenu dans l'étape (iii) pour obtenir un éluat décoloré,
(v) la purification de l'éluat décoloré obtenu dans l'étape (iv) par une nanofiltration sur des membranes ayant un seuil de rétention de 100 à 1000 g/mol, éventuellement en mode diafiltration, pour obtenir un éluat décoloré et purifié, et
(vi) la concentration de l'éluat décoloré et purifié obtenu dans l'étape (v) pour obtenir un concentrat contenant l'acide aconitique.

Dans un autre mode de réalisation particulier, le procédé de l'invention comprend :
(i) la clarification d'une solution d'un produit issu de l'industrie de la canne à sucre pour obtenir une solution clarifiée contenant l'acide aconitique, et
(ii) la mise en contact de ladite solution clarifiée avec une résine faiblement ou fortement anionique sous forme de base libre ou préalablement conditionnée sous forme d'un contre-anion,
(iii) l'élution de l'acide aconitique à partir de ladite résine pour obtenir un éluât contenant l'acide aconitique,
(iv) la purification de l'éluat obtenu dans l'étape (iii) par une nanofiltration sur des membranes ayant un seuil de rétention de 100 à 1000 g/mol, éventuellement en mode diafiltration, pour obtenir un éluat décoloré et purifié,
(v) la décoloration de l'éluat purifié obtenu dans l'étape (iv) pour obtenir un éluat décoloré et purifié, et
(vi) la concentration de l'éluat décoloré et purifié obtenu dans l'étape (v) pour obtenir un concentrat contenant l'acide aconitique.

Dans un autre mode de réalisation particulier, le procédé de l'invention comprend :
(i) la clarification d'une solution d'un produit issu de l'industrie de la canne à sucre pour obtenir une solution clarifiée contenant l'acide aconitique, et
(ii) la mise en contact de ladite solution clarifiée avec une résine faiblement ou fortement anionique sous forme de base libre ou préalablement conditionnée sous forme d'un contre-anion,
(iii) l'élution de l'acide aconitique à partir de ladite résine pour obtenir un éluât contenant l'acide aconitique,
(iv) la purification de l'éluat obtenu dans l'étape (iii) par une nanofiltration sur des membranes ayant un seuil de rétention de 100 à 1000 g/mol, éventuellement en mode diafiltration, pour obtenir un éluat décoloré et purifié,
(v) la concentration de l'éluat purifié obtenu dans l'étape (iv) pour obtenir un concentrat contenant l'acide aconitique, et
(vi) la décoloration du concentrat obtenu dans l'étape (v) pour obtenir un concentrat décoloré.

Un autre mode de réalisation particulier de l'invention concerne un procédé d'extraction comprenant en outre une étape de cristallisation du concentrat ou d'une solution décolorée, ladite solution décolorée pouvant être soit l'éluat décoloré, soit l'éluat purifié et décoloré, soit le concentrat décoloré.

L'acide aconitique cristallisé peut être récupéré par toutes techniques connues de l'homme du métier, telle que la filtration sous vide. La fraction liquide colorée résiduelle peut être recyclée soit en nanofiltration, soit en fixation sur résine.

Dans un mode de réalisation avantageux de l'invention, le procédé d'extraction de l'acide aconitique comprend :
(i) la mise en solution d'un produit issu de l'industrie de la canne à sucre, et éventuellement la clarification de ladite solution,
(ii) la mise en contact de la solution obtenue dans l'étape (i) avec une résine faiblement ou fortement anionique sous forme de base libre ou préalablement conditionnée sous forme d'un contre-anion,
(iii) l'élution par une solution d'un acide ou d'une base de l'acide aconitique à partir de ladite résine pour obtenir un éluat contenant de l'acide aconitique,
(iv) la purification de l'éluat obtenue dans l'étape (iii) par une nanofiltration sur des membranes ayant un seuil de rétention de 100 à 1000 g/mol pour obtenir une solution purifiée contenant de l'acide aconitique,
(v) éventuellement, la concentration de la solution purifiée obtenue dans l'étape (iv) pour obtenir un concentrat contenant de l'acide aconitique,
(vi) éventuellement, la décoloration de l'éluat obtenu dans l'étape (iii) ou l'éluat purifié obtenu dans l'étape (iv) ou du concentrat obtenu dans l'étape (v) pour obtenir une solution décolorée contenant de l'acide aconitique,
(vii) la cristallisation soit de l'éluat purifié obtenu dans l'étape (iv), soit du concentrat obtenu dans l'étape (v), soit du concentrat décoloré obtenu dans l'étape (vi) pour obtenir l'acide aconitique cristallisé, et
(viii) le séchage de l'acide aconitique cristallisé à l'étape (vii).

Le séchage de l'acide aconitique peut être réalisé par toutes techniques de séchage à l'échelle industrielle connues de l'homme du métier, telles que la technique d'atomisation.

Selon un mode de réalisation particulièrement avantageux, le procédé d'extraction de l'invention comprend :
(i) la mise en solution de la mélasse ou de la vinasse, et éventuellement la clarification de ladite solution, par microfiltration utilisant des membranes ayant une porosité de 0,1 à 10 µm, suivie par une ultrafiltration utilisant des membranes ayant une porosité de 1 à 100 nm,
(ii) la mise en contact de la solution obtenue dans l'étape (i) avec une résine faiblement anionique, avantageusement comportant des groupements d'amine tertiaire, conditionnée sous forme d'un contre-anion, avantageusement le sulfate,
(iii) l'élution par une solution d'acide sulfurique, avantageusement à une concentration de 2N, de l'acide aconitique à partir de ladite résine pour obtenir un éluat contenant de l'acide aconitique,
(iv) la purification de l'éluat obtenu dans l'étape (ii) par une nanofiltration en mode diafiltration sur des membranes ayant un seuil de rétention de 100 à 1000 g/mol pour obtenir un éluat purifié contenant de l'acide aconitique,
(v) la concentration de l'éluat purifié obtenu dans l'étape (iv) par osmose inverse suivie d'une nanofiltration et d'une évaporation pour obtenir un concentrat contenant de l'acide aconitique,
(vi) la décoloration du concentrat obtenu dans l'étape (v) par charbon actif pour obtenir un concentrat décoloré contenant de l'acide aconitique,
(vii) la filtration du concentrat décoloré obtenu dans l'étape (vi) pour éliminer le charbon actif du concentrat décoloré,
(viii) la cristallisation du concentrat décoloré obtenu dans l'étape (vii) pour obtenir l'acide aconitique cristallisé,
(ix) le séchage de l'acide aconitique cristallisé dans l'étape (viii).

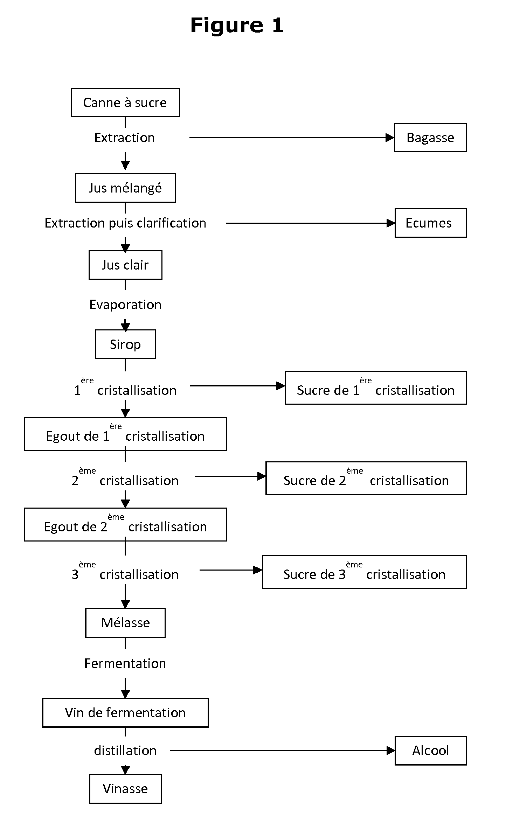

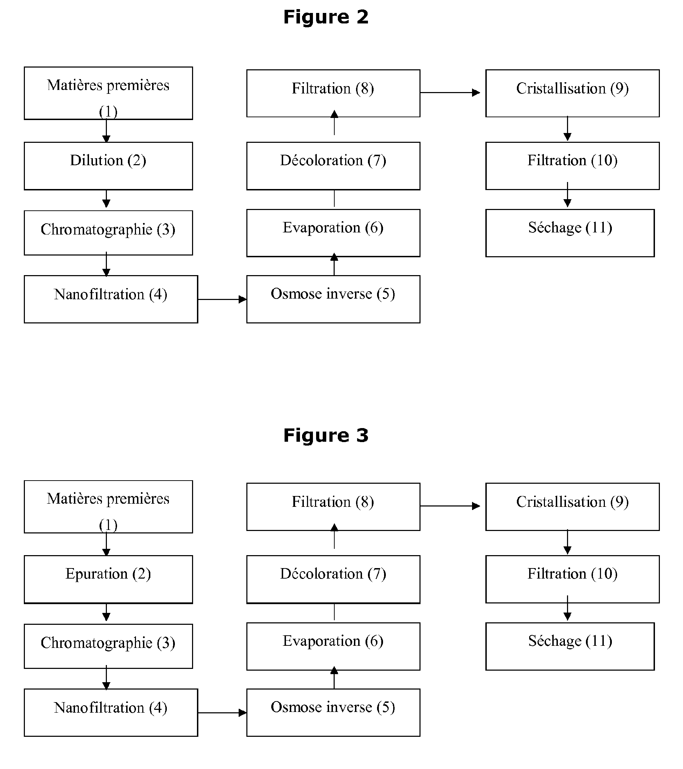

Les figures 1 à 6 et les exemples 1 et 2 à qui suivent illustrent certains modes de réalisation de l'invention.

La figure 1 représente un schéma de traitement de la canne à sucre.

La figure 2 illustre le schéma du procédé d'extraction de l'acide aconitique à partir de mélasse de la canne à sucre. Les flèches représentent l'enchaînement des étapes.

La figure 3 illustre le schéma du procédé d'extraction de l'acide aconitique à partir de vinasse de la canne à sucre. Les flèches représentent l'enchaînement des étapes.

La figure 4 illustre la courbe de percée des acides organiques de la vinasse microfiltrée. L'axe des abscisses correspond au volume de l'effluent de sortie de colonne (BV). L'axe des coordonnées correspond à la concentration massique relative.

La figure 5 représente la courbe d'élution de l'acide aconitique et des impuretés après saturation de la résine par de la vinasse microfiltrée. L'axe des abscisses correspond au volume de l'éluat (BV). L'axe des ordonnées correspond à la concentration de sulfate, d'aconitate, ou de chlorure dans l'éluat.

La figure 6 représente la courbe d'élution de l'acide aconitique et des impuretés après saturation de la résine par de la mélasse diluée. L'axe des abscisses correspond au volume de l'éluat (BV). L'axe des ordonnées correspond à la concentration de sulfate, d'aconitate, ou de chlorure dans l'éluat.

### Exemple 1 : Extraction de l'acide aconitique à partir de vinasse

### 1.1 Clarification de vinasse

La vinasse est microfiltrée sur membranes céramiques ayant une porosité de 0,13 µm. La pression relative appliquée à la membrane est de 0,13 MPa et le débit de perméat est alors de 100 L/h. Au cours de la microfiltration, la température du rétentat et du perméat évolue de 45 à 70°C. La microfiltration est arrêtée à un facteur de concentration volumique est de 9,3.

### 1.2 Chromatographie sur colonne

### 1.2.1 Préparation de la colonne chromatographique

La résine Lewatit S4528 est conditionnée à l'acide sulfurique à 1 N à un débit de 5 BV/h puis rincée à l'eau à un débit de 2,5 BV/h jusqu'à atteindre un pH égal à celui de l'alimentation (vinasse microfiltrée).

### 1.2.2 Fixation

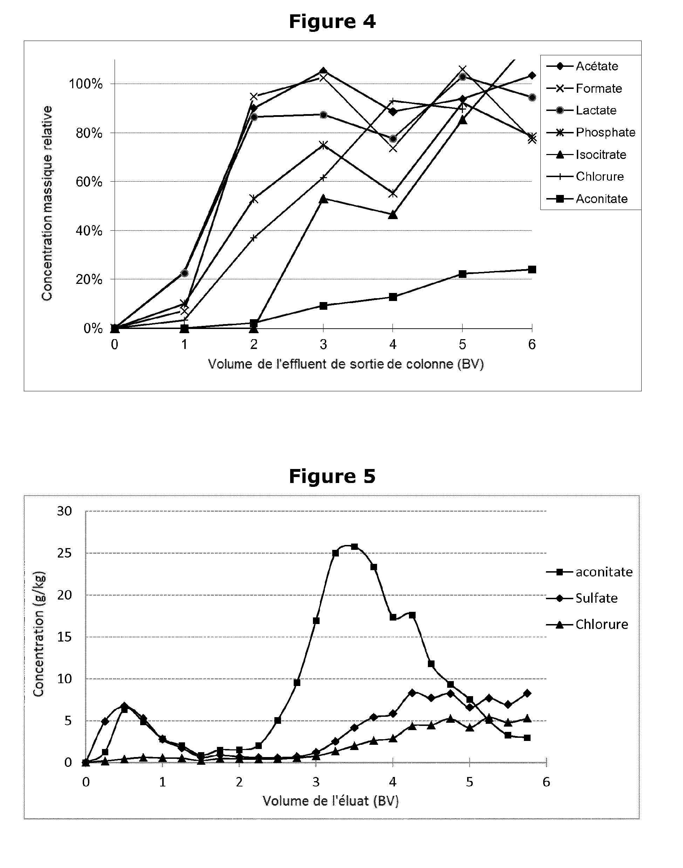

La vinasse microfiltrée est percolée sur le lit de résine, qui retient préférentiellement l'acide aconitique (figure 4), à un débit de 5 BV/h.

### 1.2.3 Elution

Les composés sont élués à l'acide sulfurique à 0,5 N à un débit de 1 BV/h. La fraction collectée de 2,25 à 3,25 BV constitue l'éluat d'intérêt (figure 5). Le sulfate est l'impureté majoritaire dans l'éluat d'intérêt avec un ratio massique acide aconitique/sulfate de 10,4. La manipulation a été répétée trois fois afin d'obtenir un éluat d'intérêt de volume total égal à 3 BV, soit 30 L. L'absorbance mesurée à 420 nm (longueur d'onde caractéristique des colorants de sucrerie) de l'éluat d'intérêt dilué 100 fois est de 0,89, soit un indice de couleur de 0,89 x 100 x 30 = 2670.

### 1.3 Nanofiltration

L'éluat d'intérêt est nanofiltré en mode de diafiltration sur des membranes organiques dont le seuil de coupure est de 150-300 g/mol. La pression appliquée est comprise entre 1,5 et 2,0 MPa. Le volume de perméat récupéré est de 800 L. Le perméat final présente un ratio massique acide aconitique / sulfate de 26,4. L'absorbance mesurée à 420 nm du perméat de nanofiltration non dilué est de 0,15, soit un indice de couleur de 0,15 x 1 x 800 = 120. La nanofiltration a donc permis de purifier la solution d'acide aconitique en éliminant partiellement le sulfate et les colorants.

### 1.4 Osmose inverse

Le perméat issu de la nanofiltration est concentré en osmose inverse sur des membranes organique (taux de rétention du NaCl de 99,5%) à 20 °C et à une pression de 1,2 MPa. La concentration en acide aconitique est alors multipliée par 58.

### 1.5 Evaporation, Cristallisation et Décoloration

Le rétentat issu de l'osmose inverse est concentré par évaporation puis décoloré par charbon actif. L'acide aconitique contenu dans la solution décolorée est alors cristallisé. Les cristaux humides décolorés sont séchés sous vide.

La poudre obtenue en fin de procédé contient 95,3 grammes d'acide aconitique pour 100 grammes de poudre sèche.

### Exemple 2 : Extraction de l'acide aconitique à partir de mélasse

### 2.1 Dilution de la mélasse brute

La mélasse brute est diluée 8 fois (m/m) avec de l'eau.

### 2.2 Chromatographie sur colonnes

### 2.2.1 Préparation de la colonne chromatographique

La résine S4528 est conditionnée à l'acide sulfurique 1 N à un débit de 2,5 BV/h jusqu'à pH 1 puis rincée à l'eau à un débit de 5 BV/h jusqu'à pH 2.

### 2.2.2 Fixation de l'acide aconitique

La mélasse diluée est percolée sur le lit de résine, qui retient préférentiellement l'acide aconitique à un débit de 5 BV/h.

### 2.2.3 Elution de l'acide aconitique

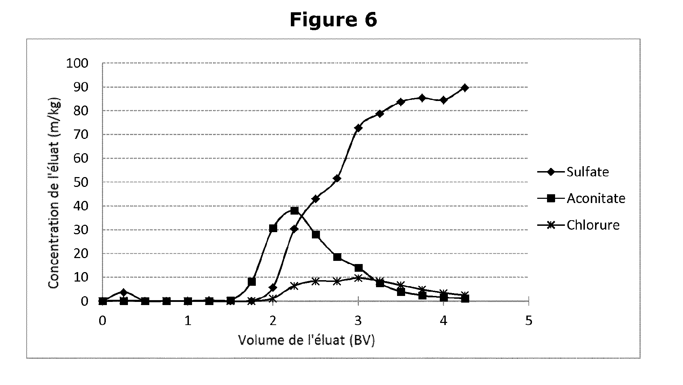

Les composés sont élués à l'acide sulfurique à 0,5 N à un débit de 2 BV/h. La fraction collectée de 1,5 à 2,5 BV constitue l'éluat d'intérêt (la figure 6). Le sulfate est l'impureté majoritaire dans l'éluat d'intérêt avec un ratio massique acide aconitique / sulfate de 2,3.

### 2.3 Nanofiltration

L'éluat d'intérêt est nanofiltré en mode de diafiltration sur des membranes organiques (NFX 2B 2540) dont le seuil de coupure est de 150-300 g/mol. La pression appliquée est comprise entre 1,5 et 2,0 MPa. Le perméat final présente un ratio massique acide aconitique / sulfate de 25,0. La nanofiltration a donc permis de purifier la solution d'acide aconitique en éliminant partiellement le sulfate.

### 2.4 Osmose inverse

Le perméat issu de la nanofiltration est concentré en osmose inverse sur des membranes organique (taux de rétention du NaCl de 99,5%) à 20 °C et à une pression de 1,2 MPa. La concentration en acide aconitique est alors multipliée par 54.

### 2.5 Décoloration et cristallisation

Le rétentat issu de l'osmose inverse est concentré par évaporation puis décoloré par charbon actif. L'acide aconitique contenu dans la solution décolorée est alors cristallisé. Les cristaux humides décolorés sont séchés sous vide.

La poudre obtenue en fin de procédé contient 90,2 grammes d'acide aconitique pour 100 grammes de poudre sèche.

## Revendications

1. Procédé d'extraction de l'acide aconitique à partir de produits issus de l'industrie de la canne à sucre, ledit procédé comprenant :
(i) la mise en contact d'une solution de l'un desdits produits contenant l'acide aconitique avec une résine faiblement ou fortement anionique sous forme de base libre ou préalablement conditionnée sous forme d'un contre-anion,
(ii) l'élution de l'acide aconitique à partir de ladite résine pour obtenir un éluat contenant l'acide aconitique, et
(iii) la purification de l'éluat par une nanofiltration sur des membranes ayant un seuil de rétention de 100 à 1000 g/mol, éventuellement en mode diafiltration, pour obtenir un éluat purifié contenant l'acide aconitique,
ladite résine comportant des groupements ammonium quaternaire et des groupements amines tertiaires et ayant une granulométrie comprise entre 200 et 2000 µm.

2. Procédé d'extraction selon la revendication 1, **caractérisé en ce que** les produits issus de l'industrie de la canne à sucre sont choisis dans le groupe comprenant le jus d'extraction, le jus clair, le sirop, l'égout de 1^{ère} cristallisation, l'égout de 2^{ème} cristallisation, l'égout de 3^{ème} cristallisation ou mélasse, les vins de fermentation obtenus à partir des produits précédemment cités et les vinasses obtenues après distillation de ces vins.

3. Procédé d'extraction selon la revendication 1 ou 2, **caractérisé en ce que** ledit procédé comprend en outre une étape de clarification par filtration ou centrifugation du susdit produit contenant l'acide aconitique avant l'étape de mise en contact d'une solution dudit produit avec ladite résine faiblement ou fortement anionique.

4. Procédé d'extraction selon la revendication 3, **caractérisé en ce que** l'étape de clarification est mise en oeuvre par une filtration utilisant des membranes ayant une porosité de 0,1 à 10 µm, éventuellement suivie par une filtration utilisant des membranes ayant une porosité de 1 à 100 nm.

5. Procédé d'extraction selon l'une quelconque des revendications précédentes, **caractérisé en ce que** ledit procédé comprend en outre une étape de concentration de l'acide aconitique en aval de la purification de l'éluat, ladite étape de concentration étant éventuellement mise en oeuvre par osmose inverse et/ou nanofiltration, éventuellement suivi par évaporation, pour obtenir un concentrat contenant l'acide aconitique.

6. Procédé d'extraction selon les revendications 3-5, **caractérisé en ce que** ledit procédé comprend en outre une étape de décoloration en aval de la clarification d'un produit issu de l'industrie de la canne à sucre contenant l'acide aconitique, pour obtenir une solution décolorée contenant l'acide aconitique.

7. Procédé d'extraction selon l'une quelconque des revendications 1 à 6, **caractérisé en ce que** ledit procédé comprend en outre une étape de cristallisation de l'éluat purifié du concentrat ou d'une solution décolorée pour obtenir l'acide aconitique solide, et éventuellement une étape de séchage dudit acide aconitique solide.

8. Procédé d'extraction selon l'une quelconque des revendications précédentes, **caractérisé en ce que** l'acide aconitique obtenu par ledit procédé est sous forme *cis,* sous forme *trans* ou sous un mélange de ces deux formes.

9. Procédé d'extraction selon l'une quelconque des revendications précédentes, **caractérisé en ce que** ledit procédé comprend :
(i) la mise en solution d'un produit issu de l'industrie de la canne à sucre, et éventuellement la clarification de ladite solution,
(ii) la mise en contact de la solution obtenue dans l'étape (i) avec une résine faiblement ou fortement anionique sous forme de base libre ou préalablement conditionnée sous forme d'un contre-anion,
(iii) l'élution par une solution d'un acide ou d'une base de l'acide aconitique à partir de ladite résine pour obtenir un éluat contenant de l'acide aconitique,
(iv) la purification de l'éluat obtenue dans l'étape (iii) par une nanofiltration sur des membranes ayant un seuil de rétention de 100 à 1000 g/mol pour obtenir un éluat purifiée contenant de l'acide aconitique,
(v) éventuellement, la concentration de la solution purifiée obtenue dans l'étape (iv) pour obtenir un concentrat contenant de l'acide aconitique,
(vi) éventuellement, la décoloration de l'éluat obtenu dans l'étape (iii) ou l'éluat purifié obtenu dans l'étape (iv) ou du concentrat obtenu dans l'étape (v) pour obtenir une solution décolorée contenant de l'acide aconitique,
(vii) la cristallisation soit de l'éluat purifié obtenu dans l'étape (iv), soit du concentrat obtenu dans l'étape (v), soit du concentrat décoloré obtenu dans l'étape (vi) pour obtenir l'acide aconitique cristallisé, et
(viii) le séchage de l'acide aconitique cristallisé à l'étape (vii).

10. Procédé d'extraction selon la revendication 9, **caractérisé en ce que** l'étape d'élution est réalisée par une solution d'acide à une concentration comprise entre 0,1 et 6 N, ledit acide étant choisi parmi l'acide sulfurique ou l'acide chlorhydrique ou par une solution de base à concentration comprise entre 0,1 et 6 N, ladite base étant choisie parmi l'hydroxyde de sodium ou l'hydroxyde de potassium.

11. Procédé d'extraction selon l'une quelconque des revendications précédentes, **caractérisé en ce que** le débit de fixation d'une solution contenant l'acide aconitique est de 0,5 à 20 BV/h, notamment de 1 à 5 BV/h, et que le débit d'élution est de 0,5 à 10 BV/h, notamment de 0,5 à 5 BV/h.

12. Procédé d'extraction selon l'une quelconque des revendications précédentes, **caractérisé en ce que** ledit procédé comprend :
(i) la mise en solution de la mélasse ou de la vinasse, et éventuellement la clarification de ladite solution, par microfiltration utilisant des membranes ayant une porosité de 0,1 à 10 µm, suivie par une ultrafiltration utilisant des membranes ayant une porosité de 1 à 100 nm,
(ii) la mise en contact de la solution obtenue dans l'étape (i) avec une résine faiblement anionique conditionnée sous forme d'un contre-anion, avantageusement le sulfate,
(iii) l'élution par une solution d'acide sulfurique de l'acide aconitique à partir de ladite résine pour obtenir un éluat contenant de l'acide aconitique,
(iv) la purification de l'éluat obtenu dans l'étape (ii) par une nanofiltration en mode diafiltration sur des membranes ayant un seuil de rétention de 100 à 1000 g/mol pour obtenir un éluat purifié contenant de l'acide aconitique,
(v) la concentration de l'éluat purifié obtenu dans l'étape (iv) par osmose inverse suivie d'une nanofiltration et d'une évaporation pour obtenir un concentrat contenant de l'acide aconitique,
(vi) la décoloration du concentrat obtenu dans l'étape (v) par charbon actif pour obtenir un concentrat décoloré contenant de l'acide aconitique,
(vii) la filtration du concentrat décoloré obtenu dans l'étape (vi) pour éliminer le charbon actif du concentrat décoloré,
(viii) la cristallisation du concentrat décoloréobtenu dans l'étape (vii) pour obtenir l'acide aconitique cristallisé,
(ix) le séchage de l'acide aconitique cristallisé dans l'étape (viii).
